# EUROPEAN PATENT SPECIFICATION

(11) **EP 1 185 313 B1**
(45) Date of publication and mention of the grant of the patent: **02.02.2005**
(21) Application number: 00936307.8
(22) Date of filing: 25.05.2000
(51) Int. Cl.: A61L 27/16, A61L 27/56

(54) **EXPANDED POLYTETRAFLUOROETHYLENE VASCULAR GRAFT WITH INCREASED HEALING RESPONSE**
GEFÄSSTRANSPLANTAT AUS EXPANDIERTEM POLYTETRAFLUOROETHYLEN MIT ERHÖHTER HEILUNG
GREFFON VASCULAIRE EN POLYTETRAFLUORETHYLENE EXPANSE A REACTION DE CICATRISATION AMELIOREE

(30) Priority: 26.05.1999 US 320383
(43) Date of publication of application: 13.03.2002
(73) Proprietor: BARD PERIPHERAL VASCULAR, INC., Tempe, AZ 85280-1740 (US)
(72) Inventor: EDWIN, Tarun, J., Tempe, AZ 85282 (US); BANAS, Christopher, E., San Antonio, TX 78231 (US)
(74) Representative: HOFFMANN - EITLE
(86) International application number: PCT/US2000/014453
(87) International publication number: WO 2000/071179

(56) References cited:
- US-A- 5 800 512
- US-A- 5 861 033

## Description

### BACKGROUND OF THE INVENTION

### 1. Field of the Invention

The present invention relates generally to synthetic implantable vascular prostheses which are suitable for implantation into a human body for a wide variety of applications, including, without limitation, bypass applications or arterio-venous access grafting. More particularly, the present invention relates to implantable vascular grafts fabricated from expanded polytetrafluoroethylene.

### 2. Background and Description of Related Art

Because of its biocompatible properties many types of surgical implants and prostheses have been made of polytetrafluoroethylene (PTFE). Successful vascular grafts are formed from expanded polytetrafluoroethylene (ePTFE), which is characterized by a microporous structure consisting of "nodes" interconnected by "fibrils." Expanded PTFE tubular products are formed by admixing PTFE resin particles with an extrusion lubricant (e.g. mineral spirits) to form a slurry. This slurry is then compacted into a cylindrical extrusion billet, which is placed in a ram extruder and extruded through a die to form a tubular extrudate. The tubular extrudate is then dried, i.e., heated to evaporate the lubricant. As might be expected from material formed from compacted resin particles, the resulting extrudate has rather limited longitudinal and radial tensile strength.

The dried tubular extrudate is then longitudinally expanded by longitudinally stretching while being heated to a temperature below the crystalline melting point of PTFE. Longitudinal expansion of the tubular extrudate causes fibrils of PTFE to be pulled from the nodes of coalesced resin particles. These fibrils are oriented parallel to the axis of expansion and interconnect adjacent nodes. The length of the fibrils, that is the distance between the nodes, is know as the internodal distance or "IND". As used herein, the term IND is intended to mean the distance measured between closest opposing surfaces of adjacent nodes measured along the longitudinal axis of a fibril. INDs are derived by randomly measuring five internodal distances and averaging the sum of the five internodal distances. The measurements are expressed in µms (10⁻⁶ meters).

After longitudinal expansion, the ePTFE exhibits increased longitudinal tensile strength, but still lacks adequate radial tensile strength. The longitudinally expanded tubular extrudate is next "sintered" by heating to a temperature above the crystalline melting point of the PTFE for a period sufficient to "melt" a portion of the crystalline material into the amorphous state. This process stabilizes or "fixes" the structural properties of ePTFE to prevent subsequent shrink back of the expanded material. Sintered ePTFE exhibits significantly increased longitudinal tensile strength as well as radial tensile strength relative to the unsintered material.

Conventional ePTFE vascular grafts, such as those made by W. L. Gore & Associates, Inc. consist of an ePTFE tube having average IND of 10-40 µm These grafts have an external circumferential reinforcing wrap of thin film ePTFE tape. Conventional grafts made by IMPRA, Inc. (a subsidiary of C.R. Bard, Inc.) also have average IND of 10-40 µm but consist of a monolithic tube of ePTFE without a reinforcing wrap. Conventional grafts are commonly used to replace lengths of diseased vascular tissue by suturing the ePTFE grafts to the patient's vasculature to bypass the diseased tissue. Because PTFE is biocompatible there is very little immune reaction against these foreign bodies. However, the PTFE surface does stimulate some platelet binding and activation with related clot formation. Many of the conventional ePTFE grafts fail over a period of time (poor patency of the graft), and a common failure mode is one of occlusion by a blood clot (thrombus) within the graft lumen

The conventional grafts fail to exhibit a complete healing response *in vivo*. See, e.g., Clowes, A.W., et al., "Mechanisms of arterial graft failure. II. Chronic endothelial and smooth muscle cell proliferation in healing polytetrafluoroethylene prostheses," *J*. *Vasc*. *Surg.;* 3:877-884 (1986); Golden, M.A., et al., "Healing of polytetrafluoroethylene arterial grafts is influenced by graft porosity," *J*. *Vasc*. *Surg.;* 11:838-845 (1990); and "Influence of fibril length upon ePTFE graft healing and host modification of the implant," *J*. *Of Biomed*. *Materials Res.* 26:1422-1447 (1992). As used herein, the term "healing response" is intended to mean the ability of an ePTFE graft to develop luminal endothelial cell coverage of substantially uniform thickness along the longitudinal axis of the luminal wall surface of the graft (this cell layer is termed the intima or neointima). The healing response is significant because the intimal cell layer is non-thrombogenic and resists or even reverses the formation of thrombus.

It has been found that conventional ePTFE interposition vascular grafts, i.e., those with internodal distances of 10-40 µm exhibit *in vivo* endothelialization from the anastomotic sites which progresses toward the interanastomotic region of the graft along the lumen of the graft, without exhibiting transmural capillary ingrowth. The endothelialization of anastomotic site origin generally fails to cover the luminal surface of all but the shortest grafts with a neointimal cell layer sufficient to prevent thrombus formation within the graft This result leads invariably to decreased graft patency due to thrombosis of the graft requiring eventual replacement of the failed graft.

However, in the above-cited scientific literature and references cited therein it has been demonstrated that porosity of ePTFE influences the healing response of grafts. In some studies, more porous (larger IND) grafts have shown improved healing, but this result has been somewhat variable. It is generally agreed that grafts with small INDs (e.g. under about 40 µm) show poor healing; however, some studies show grafts with large INDs (e.g., over 60 µm) also heal poorly. It is also known that increasing the porosity of ePTFE markedly decreases tensile strength and other favorable physical properties of the graft.

U.S. Patent 5,800,512 to **Lentz et al**. discloses a compound ePTFE graft with improved healing properties. In this disclosure a first tubular extrudate is expanded on a mandrel to an IND of between 40 and 100 µm. Then a second extrudate is expanded over the mandrel-borne first extrudate. The diameter of the second extrudate is selected so that its final expansion is less than that of the first extrudate. After expansion the IND of the second extrudate is between 15 and 35 µm. The two extrudates are then bonded together by sintering. This compound structure was necessary because the radial tensile strength of the inner layer (large IND) alone is only about 0.2 kg/mm² while that of the compound structure is identical to that of the outer (small IND) extrudate alone―about 0.48 kg/mm². Although the inventors report improved healing with this composite graft, it would appear that the relatively small INDs of the outer layer of the structure would preclude transmural capillary growth that has been postulated to be an important factor in complete healing of ePTFE grafts.

US 5861033 discloses an ePTFE graft with inwardly tapered pores.

### OBJECTS AND SUMMARY OF THE INVENTION

The present invention provides an improved ePTFE vascular graft suitable for interposition or bypass applications which exhibits an improved *in vivo* healing response characterized by transmural capillary ingrowth which supports complete endothelialization and smooth muscle cell growth along the entire luminal surface of the graft. The inventive improved ePTFE vascular grafts have average internodal distances in the range of 55 µm to 70 µm with a microstructure characterized by elongated columnar nodes which form substantially continuous fibrillated preclotted internodal spaces between the luminal and abluminal surface of the graft. The greater internodal distances than conventional ePTFE vascular grafts when combined with the columnar node structure afford an improved healing response *in vivo,* while maintaining at acceptable levels other physical properties of the graft, e.g., wall thickness, luminal diameter, longitudinal tensile strength, radial tensile strength, suture retention strength, etc., as conventional monolithic ePTFE grafts without a reinforcing layer which could compromise the healing response.

### BRIEF DESCRIPTION OF THE DRAWINGS

The objects and features of the present invention, which are believed to be novel, are set forth with particularity in the appended claims. The present invention, both as to its organization and manner of operation, together with further objects and advantages, may best be understood by reference to the following description, taken in connection with the accompanying drawings.
Figure 1 is a diagrammatic representation of endothelialization of a standard ePTFE graft illustrating endothelial growth at an anastomotic site.
Figure 2 is a diagrammatic representation of endothelialization of an inventive high porosity ePTFE illustrating endothelial growth and smooth muscle cell proliferation mediated by transmural capillary ingrowth to the luminal surface of the inventive graft.
Figure 3 is a scanning electron micrograph of a prior art standard commercially available expanded polytetrafluoroethylene graft taken in longitudinal cross-section at a magnification of 100X.
Figure 4 is a scanning electron micrograph of a prior art standard commercially available expanded polytetrafluoroethylene graft taken at the inside surface at a magnification of 200X.
Figure 5 is a scanning electron micrograph of high porosity graft (ERF 1425) not having the columnar nodes of the present invention having an average IND of about 57 µm taken in longitudinal cross-section at a magnification of 200X.
Figure 6 is scanning electron micrograph of the high porosity ePTFE graft of Figure 5, taken at the luminal surface thereof at a magnification of 200X.
Figure 7 a scanning electron micrograph of the high porosity ePTFE graft of Figure 5, taken at the abluminal surface thereof at a magnification of 200X.
Figure 8 is a scanning electron micrograph of the high porosity ePTFE graft (ERF 2225) of the present invention taken in longitudinal cross-section at a magnification of 100X.
Figure 9 is a scanning electron micrograph of the inventive high porosity ePTFE graft of Figure 8 taken at the abluminal surface thereof at a magnification of 100X.
Figure 10 is a scanning electron micrograph of the inventive high porosity ePTFE graft of Figure 8 taken at the luminal surface thereof at a magnification of 100X.
Figure 11 is a scanning electron micrograph of another embodiment (ERF 1719) of the inventive high porosity ePTFE vascular graft having an average IND of about 60 µm taken in longitudinal cross-section at a magnification of 100X.
Figure 12 is scanning electron micrograph of the inventive high porosity ePTFE graft of Figure 11, taken at the luminal surface thereof at a magnification of 200X.
Figure 13 a scanning electron micrograph of the inventive high porosity ePTFE graft of Figure 11, taken at the abluminal surface thereof at a magnification of 200X.
Figure 14 a light micrograph of a cross-section paraffin section of the inventive high porosity ePTFE graft of Figure 11, following implant and healing at a magnification of 20X.

### DETAILED DESCRIPTION OF THE PREFERRED EMBODIMENTS

The following description is provided to enable any person skilled in the art to make and use the invention and sets forth the best modes contemplated by the inventors of carrying out their invention.

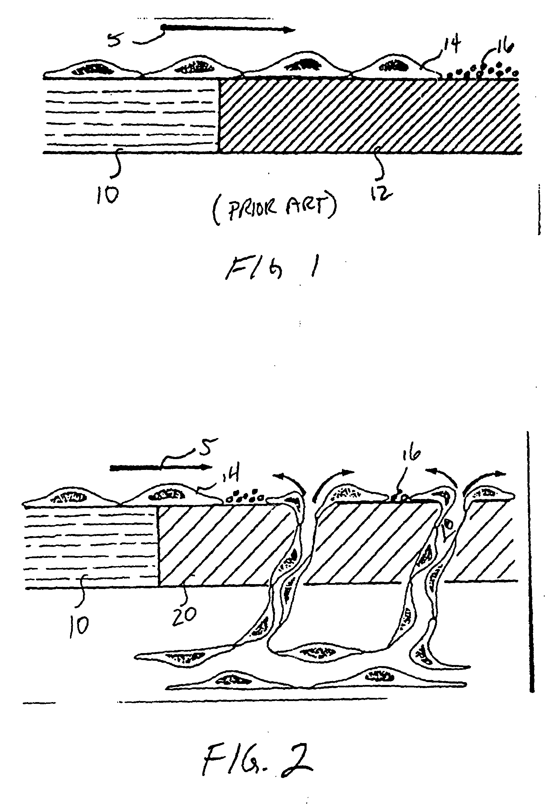

Fig. 1 shows a diagrammatic representation of a standard ePTFE vascular graft **12** anastomosed to a blood vessel **10** in accordance with the prior art. Blood flow and endothelial cell growth from the blood vessel **10** facilitate proliferation of the endothelial cells **16** and the smooth muscle cells **14** at and near the region of the anastomosis only, with no cellular migration through the wall of the vascular graft **12.** That is, cellular colonization of the graft surface occurs only by extension from the living vessel tissue at the anastomosis. In contrast, Fig. 2 depicts the healing response obtained with a high porosity ePTFE vascular graft **20** of the present invention, anastomosed to a blood vessel **10.** Here blood flow and the proliferation of endothelial cells **16** and smooth muscle cells **14** occur both along the luminal surface of the graft as well as transmurally from the aventitia through the internodal spaces **18** of the graft **20** to the luminal surface of the graft **20.** Such cellular development requires that the spaces between nodes (*i*.*e*.,. the IND) be large enough to accommodate the cellular structures and that the spaces be aligned so as to allow such cellular structures to completely penetrate the graft. The nodes are preferably oriented circumferentially.

We have found that high porosity vascular grafts, *e*.*g*., those with average internodal distances between 55 µm to 70 µm, exhibit an improved healing response when compared to standard ePTFE vascular grafts. As mentioned above, there has been some confusion in the literature over graft porosity in ePTFE grafts and improved healing characteristics. Besides mere porosity (measured as IND) it is essential for the high porosity graft to have the correct microstructure. Optimum microstructure is characterized by elongated columnar nodes which extend through a major portion of the wall thickness of the graft between the luminal and abluminal wall surfaces of the graft (*i*.*e*., essentially from one surface to the other). Such a microstructure provides essentially continuous internodal spaces for the transluminal migration of cells. Further, this structure provides improved strength over the high IND ePTFE materials made according to usual manufacturing processes. While the data herein show superior results with materials having INDs in the vicinity of 60 µm, the present invention can also produce even larger INDs (e.g., 90 µm) although there is no current evidence that such grafts exhibit healing superior to the 60 um grafts. Further, increasing levels of porosity result in loss of radial and longitudinal tensile strength. That is, inventive grafts with INDs of about 60 µm show optimal healing response combined with optimal tensile strength properties.

Preferably, the tubular vascular graft has a radial tensile strength of at least 0.3 kg/mm² and a longitudinal tensile strength of at least 0.9 kg/mm².

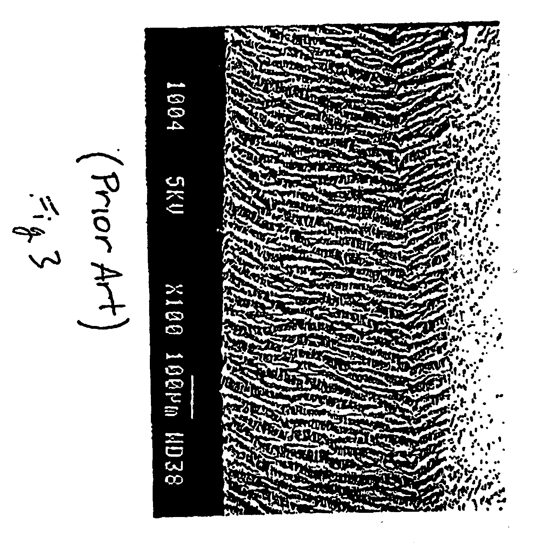

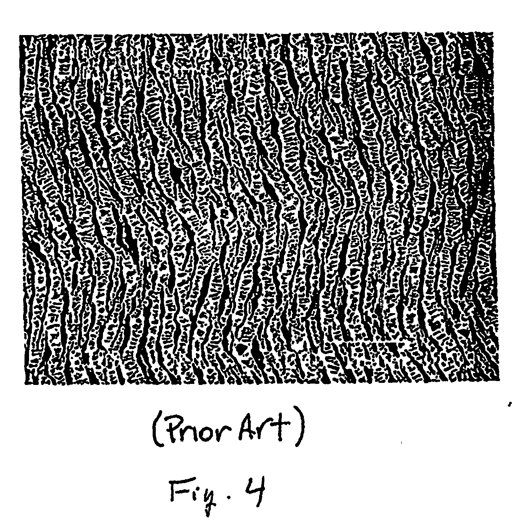

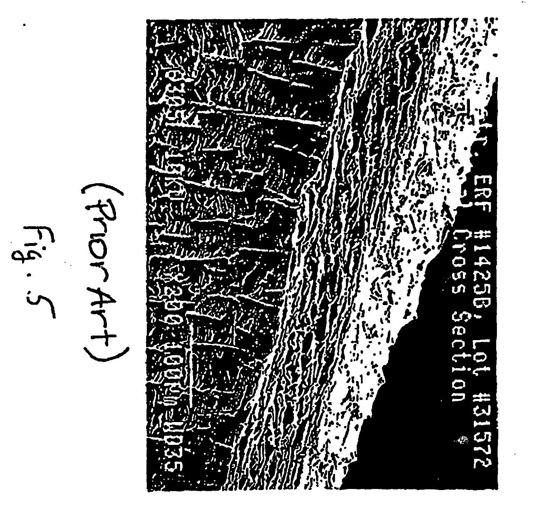

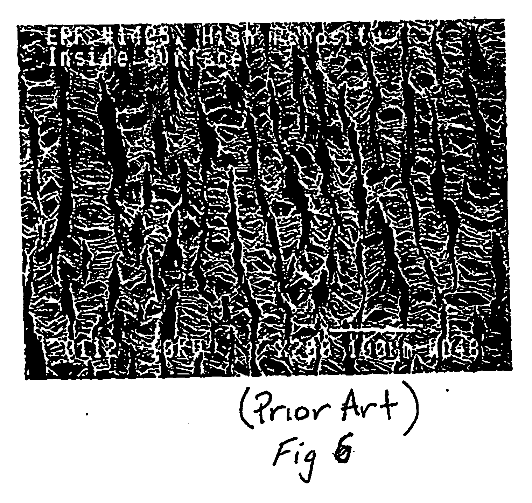

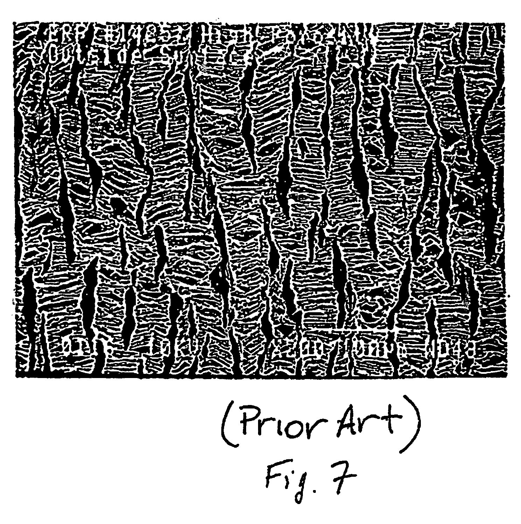

Figs. 3 through 12 are scanning electron micrographs of surfaces and longitudinal cross sections of various ePTFE grafts. Fig. 3 shows microstructure of a longitudinal cross. section of a standard 30 µm internodal distance vascular graft. Fig. 4 shows a surface view of the standard graft for comparison with the later images of the inventive graft. Note that the nodes are relatively short and irregular in shape. Neither the nodes nor the internodal spaces are continuous from surface to surface. The nodes as seen on the graft surface view are seen to be short and irregular on their ends as well as in cross-section. Figs. 5, 6 and 7 show sectional and surface views of an experimental high porosity graft produced with fairly typical manufacturing parameters except that additional expansion was used to increase the INDs to about 57 µm. Note that although the internodal spaces are larger (best seen in Figs. 6 and 7) than those of Fig. 3, the nodes are still relatively short and irregular and do not continue completely across the longitudinal cross-section (Fig. 5).

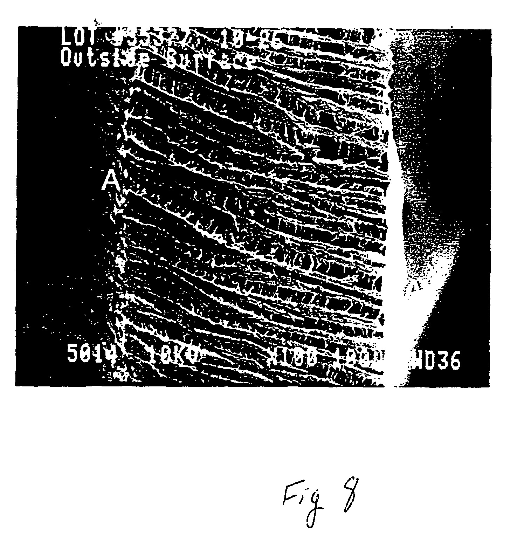

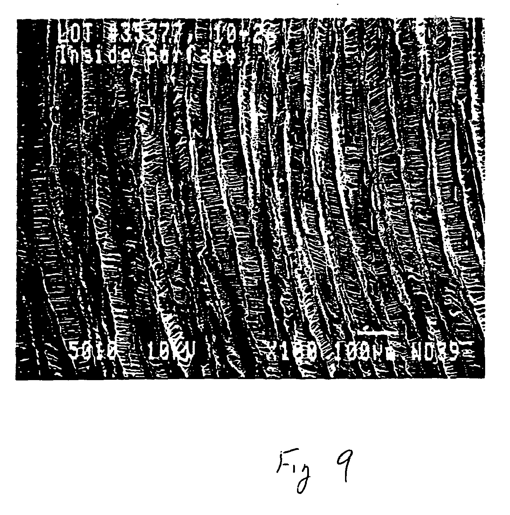

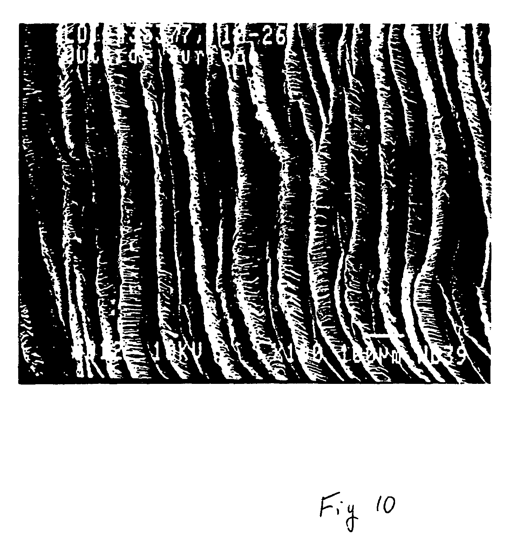

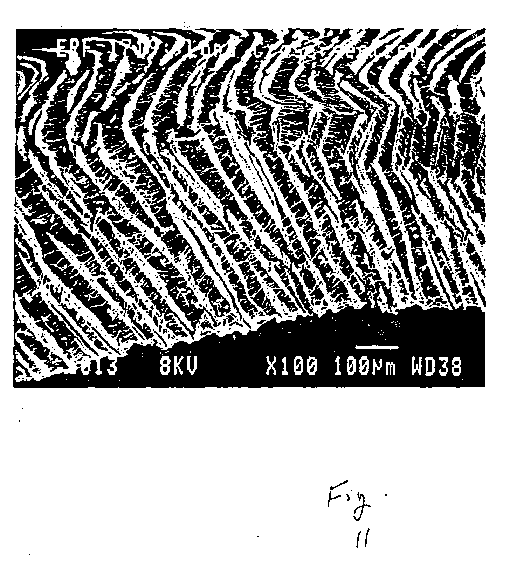

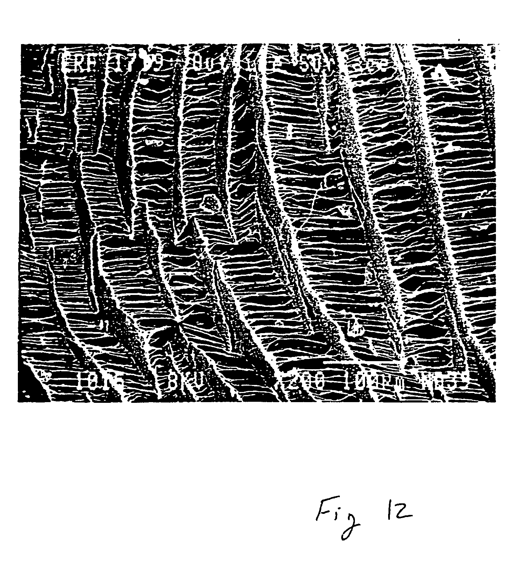

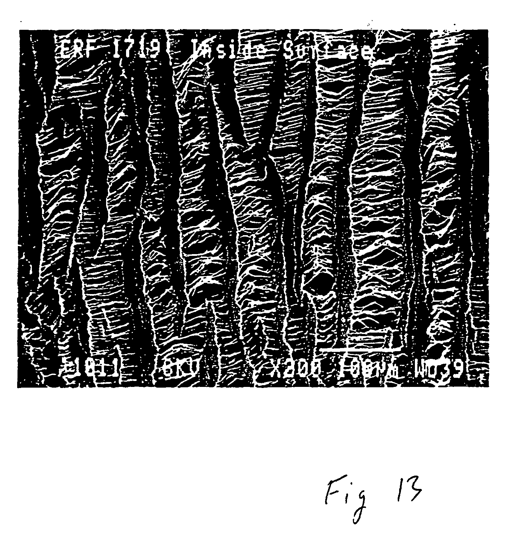

Figs 3-7 should be compared to Figs. 8-10 which show corresponding views of grafts with the columnar nodal microstructure of the inventive high porosity graft. The high porosity graft of Fig. 8 has an average internodal distance between about 52 µm to about 56 µm (from inside to outside) and exhibits the inventive columnar nodal configuration. It will be apparent to those of ordinary skill in the art that the microstructure of the standard prior art vascular graft depicted in Figs.3-7 exhibits a non-coherent nodal configuration with discontinuous internodal spaces throughout the wall thickness of the graft. On the other hand, the microstructure of the inventive high porosity graft is chafactenzed by coherent nodal configuration which forms continuous internodal spaces providing a cellular pathway between the luminal and abluminal surfaces of the graft. This is readily seen in Fig. 8 by tracing a single internodal space from one surface (at **A)** to the other surface (at **A')** of the graft. In addition Figs. 6 and 7 (the surfaces X200) should be compared to figs. 9 and 10 (magnification of X100) where the much greater length and uniformity of the nodes is readily apparent. These same characteristics are also readily apparent in Figs. 11 through 13 which shows micrographs of another sample. Here the surface views (Figs. 12 and 13) are at the same magnification as the surface views (Figs. 6 and 7) of the prior art high porosity graft that does not exhibit coherent columnar structure.

Significantly, as can be seen in Figs. 12 and 13, the nodes as viewed from the surface are long and continuous. The nodes not only extend from the luminal to the abluminal surface but have a tremendous extent in a circumferential direction. By substantially maintaining continuous coherent nodes in the circumferential direction the radial tensile strength of the inventive grafts is also increased (see Table 1, below).

Table 1 sets forth the standard physical property measurements for internal diameter (I.D.), wall thickness, radial tensile strength (RTS), longitudinal tensile strength (LTS), internodal distance (IND) for each of the luminal and abluminal surfaces, force gauge (FG) (suture retention force) and water entry pressure (WEP) for several embodiments (ERFs 1719, 1724 and 2225) of the inventive high porosity graft as well as a high porosity graft (ERF 1425) lacking the inventive coherent columnar properties of the inventive grafts. For each measurement with the exception of suture retention force, the physical properties were measured using the applicable standards promulgated by the American Association for Advancement of Medical Instrumentation (A.A.M.I.) for synthetic vascular grafts (see ANSI/AAMI VP20―1994). These measurements should be compared to physical measurements of grafts with convention ePTFE with similar INDs.

For example the 40-100 µm high porosity materials disclosed by **Lentz et al.** have an RTS (kg/mm²) of 0.2 with the "normal" graft disclosed therein having a value of 0.48. On the other hand, the coherent columnar materials disclosed herein have RTS values ranging from about 0.32 to about 0.36 (kg/mm²; 1 psi ≈ 7x10⁻⁴ kg/mm²). This illustrates how the coherent nodal structure of the present invention contributes both to cellular infiltration and radial tensile strength. ERF 1425 is also a "high porosity" graft having IND in the range of the inventive grafts but lacking coherent columnar nodes. The longitudinal and radial tensile strengths as well as the suture retention force are lower for this material as compared to the inventive coherent columnar materials.

**Table I**

| Graft | I.D. (mm) | Wall Thickness (mm) | RTS (psi) | LTS (psi) | IND luminal µm | IND ablumin al µm | FG (g) (suture retention) | WEP (psi) |
|---|---|---|---|---|---|---|---|---|
| ERF 1719 | 4 | 0.67 | 464±86.2 | 1427±63 | 61±2.9 | 62±2.9 | 1050±107 | 1.6±0.17 |
| ERF 1723 | 6 | 0.73 | 521±56.5 | 1522±22.6 | 61±1.43 | N/A | 1697±224 | 2.2±0.13 |
| ERF 2225 | 4 | 0.68 | 471±110.6 | 1338±34.9 | 52±5.4 | 56±7.4 | 1248±193.5 | 1.6±0.08 |
| ERF 1425 | 6.2 | 0.71 | 227 | 1260 | 57.5 | N/A | 886 | 1.74 |

From the foregoing, it will be appreciated that a high porosity vascular graft made of ePTFE has been described herein which exhibits average internodal distances with the range of about 52 µm to about 62 µm with a preferred microstructure characterized by elongated columnar nodes spaced apart by coherent internodal spaces occupies by fibrils. The internodal spaces have fibril densities comparable to the standard vascular graft product depicted in Fig. 3. With the exception of water entry pressure values, the inventive high porosity vascular graft exhibits physical properties comparable or superior to many commercial ePTFE graft products. Radial tensile strength, longitudinal tensile strength and suture retention strength are comparable or greater than those found in commercial products.

The grafts of the present invention have a much greater void volume (i.e., larger amount of empty space) than conventional grafts without any significant sacrifice in strength. This makes these grafts ideal for various types of delivery of drugs or other bioactive substances. It is simple to infiltrate the grafts with a variety of bioactive compounds which will then temporarily occupy the internodal spaces. In one embodiment, columnar internodal spaces are at least partially filled with non-polytetrafluoroethylene material. After the grafts are implanted the infiltrated substances will be slowly dispensed or "time released" into the blood stream and will also diffuse away from the outer surface of the graft. A variety of cellular growth factors, anti-microbials or antibiotics can be included to fight infection or to inhibitors can be added either to inhibit downstream neoplasms or to control excess smooth muscle cell proliferation during graft healing. Anti-coagulants, anti-thrombotics and antiplatelet agents can be used to limit thrombus formation until the healing process is complete. Steroidal and non steroidal anti-inflammatory drugs can aid healing. Smooth muscle contraction inhibitors, infectin ticlopidene and other active substances can enhance the effectiveness of the grafts. The present invention enables drug delivery by providing an unusual amount of empty space for the drug to occupy.

The final characteristics of ePTFE are affected by numerous manufacturing parameters as is well understood by those of skill in the art. Adequate physical and healing properties are exhibited by materials having INDs in the range of 50-70 µm and having a coherent columnar nodal structure so that both the nodes and the internodal spaces form continuous structures through the wall of the graft. It is known that amount of expansion or elongation (expressed as a percentage) influences the porosity or IND of the ePTFE. For example, **Lentz et al.** disclose that "normal porosity" (IND = 15-35 µm) is achieved with an elongation of 400% while high porosity (IND >40 µm) is achieved by an elongation of about 900%. The rate of the expansion process is also important. The desired coherent columnar structure is favored by low rates of expansion (e.g. below 100% per second). A third important factor is the ratio of lubricant to resin in formation of the extrudate. High levels of lubricant, combined with the proper rate and extent of expansion, favor the formation of the desired columnar coherent structure.

The healing response of the foregoing described high porosity vascular grafts was tested by implanting the inventive high porosity vascular grafts into four male *Papio cynocephalus* baboons. The animals were between the ages of two and three years and weighed between 11.9 and 13.2 kg at the start of the study. Standard IMPRA vascular grafts consisting of 4 mm regular wall IMPRA ePTFE grafts having an average IND of 10-40 µm were used as controls. High porosity grafts (IND of about 60 µm) that were tested had either a coherent nodal structure (the inventive grafts) or a normal nodal structure (e.g. like ERF 1425, above). The grafts were implanted following induction of anesthesia with isoflurane, and 4 mm ID high porosity or standard grafts were inserted into the common iliac circulation using previously established and standardized vascular techniques. The 60 µm high porosity grafts were preclotted with autogenous blood prior to implantation. Preclotting or other such treatment is used to limit the leakage of serum through the high porosity graft wall prior to healing.

Immediately prior to euthanasia, graft patency was confirmed by ultrasound duplex scanning. Treated animals were euthanized approximately four weeks following graft placement. Heparin (3000 units) and Evans blue (50 mg/kg) were administered by intravenous injection 1 hr before euthanasia by intravenous administration of pentobarbital. The animals were then perfused with lactated Ringer's solution at a pressure of 100 mm Hg administered by means of a thoracic aorta cannula with venous drainage from the femoral veins. Ten percent neutral buffered formalin was then perfused at the same pressure. The grafts and adjacent normal arteries were excised and immersion fixed for at least 24 hours.

Standard histological procedures were used to process the explants for light microscopic analysis. Six 33 mm segments were taken from each graft, embedded in paraffin, cross-sectioned (6 µm sections taken perpendicular to the longitudinal axis of the graft) and counterstained with hematoxylin-eosin. Sections 1 and 2 were taken from the proximal anastomosis and proximal graft. Sections 3 and 4 were taken from mid-graft, and sections 5 and 6 were taken from the distal graft and distal anastomosis.

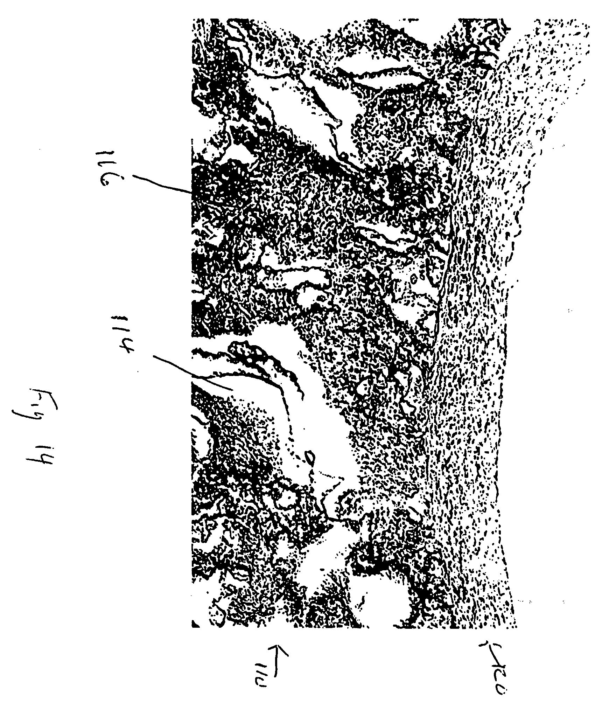

Analysis of the grafts included an evaluation of patency at the end of the study, evidence of endothelialization along the length of the graft, neointimal thickness along the length of the graft, evidence of transmural capillary ingrowth through the high porosity graft, and cellular composition in the neointimal, graft wall, and aventitia. In each of the four animals, the coherent high porosity graft (the inventive graft) exhibited a well-formed intima over most of its luminal surface. As seen in the light micrograph of Fig. 14, the neointim **120** was complete with endothelial cells. The graft matrix **110** was greatly altered in appearance. The nodal regions **114** were still visible but the internodal regions were filled with cellular material **116** (primarily smooth muscle cells and vascular channels). Neointimal formation was generally complete; the few luminal defects noted were correlated with graft regions in which the coherent nodal structure was disrupted (i.e., irregularities in fabrication). The matrix of the graft was populated by numerous smooth muscle cells and vascular capillary channels. The coherent high porosity graft exhibited luminal healing from the proximal through the distal anastomosis.

While the non-coherent high porosity graft showed somewhat better healing than the control graft, healing was not nearly as complete as in the coherent high porosity graft. In particular, healing progressed from the site of anastomosis and was not complete in the middle regions of the graft. Some regions exhibited thin layers of fibrin on the luminal surface, which was often covered by a layer of endothelial cells. The grafts often showed, particularly in the graft middle region, an open structure which appeared to be stretched and filled with fibrin. The neointima was complete only in small areas of the lumen and mural thrombi on the luminal graft surfaces indicated instability of the neointima. In some grafts large areas of acellular fibrous clot were present. No vascular channels penetrated the walls of the non-coherent high porosity grafts.

In comparison, the standard graft healed poorly with larger areas of thrombus on both surfaces. The graft structure was open and irregular and contained many regions of fibrous clot There was no neointima and only a very sparse cellular ingrowth, and that only near the ends of the grafts. Like the non-coherent high porosity graft, there was no transluminal cellular penetration.

These results indicate that high porosity (larger INDs) appears to give cells "purchase" on the luminal surface so that at least some patches of neointima can be established. However, the coherent columnar structure of the inventive grafts are necessary for establishing stable neointima and especially for complete cellular infiltration of the graft matrix including transmural vascular channels (microcapillaries).

## Claims

1. A tubular vascular graft of expanded polytetrafluoroethylene comprising:
a luminal surface spaced apart from an abluminal surface, wherein said surfaces are connected by coherent columnar nodes of polytetrafluoroethylene which are separated by preclotted columnar internodal spaces; and
fibrils of polytetrafluoroethylene having an average length between 55 and 70 µm between the luminal and abluminal surfaces, spanning each preclotted columnar internodal space to connect each columnar node with an immediately adjacent columnar node.

2. The tubular vascular graft of Claim 1 having a radial tensile strength of at least 0.3 kg/mm².

3. The tubular vascular graft of Claim 1 having a longitudinal tensile strength of at least 0.9 kg/mm².

4. The tubular vascular graft of Claim 1, wherein columnar internodal spaces are at least partially filled by non-polytetrafluoroethylene material.

5. The tubular vascular graft of Claim 4, wherein said non-polytetrafluoroethylene material is selected from the group consisting of antibiotics, antimicrobial agents, antineoplastic agents, smooth muscle inhibitors, antithrombic agents, antiplatelet agents, non-steroidal anti-inflammatory agents, collagen, anti-angiogenesis agents, anticoagulants, and cellular growth factors.

6. The tubular vascular graft of Claim 1, wherein the fibrils are oriented parallel to a longitudinal axis of the tubular graft.

7. The tubular vascular graft of Claim 1, wherein said nodes are oriented circumferentially.

## Patentansprüche

1. Röhrenförmiger vaskulärer Graft aus expandiertem Polytetrafluorethylen, umfassend:
eine luminale Oberfläche, die von einer abluminalen Oberfläche beabstandet ist, worin die Oberflächen durch zusammenhängende säulenförmige Knoten aus Polytetrafluorethylen verbunden sind, die durch vorkoagulierte säulenförmige Zwischenknotenräume getrennt sind, verbunden sind;
Fibrillen aus Polytetrafluorethylen mit einer durchschnittlichen Länge zwischen 55 und 70 µm zwischen den luminalen und abluminalen Oberflächen, die jeden vorkoagulierten säulenförmigen Zwischenknotenraum überspannen, um jeden säulenförmigen Knoten mit einem unmittelbar benachbarten säulenförmigen Knoten zu verbinden.

2. Röhrenförmiger vaskulärer Graft gemäss Anspruch 1 mit einer radialen Zugfestigkeit von wenigstens 0,3 kg/mm².

3. Röhrenförmiger vaskulärer Graft gemäss Anspruch 1 mit einer longitudinalen Zugfestigkeit von wenigstens 0,9 kg/mm².

4. Röhrenförmiger vaskulärer Graft gemäss Anspruch 1, worin die säulenförmigen Zwischenknotenräume zumindest teilweise mit einem Nicht-Polytetrafluorethylenmaterial gefüllt sind.

5. Röhrenförmiger vaskulärer Graft gemäss Anspruch 4, worin das Nicht-Polytetrafluorethylenmaterial aus der Gruppe ausgewählt ist, die aus Antibiotika, antimikrobiellen Mitteln, antineoplastischen Mitteln, Inhibitoren der glatten Muskulatur, Antithrombotika, Antiblutplättchenmitteln, nicht-steroidalen entzündungshemmenden Mitteln, Kollagen, Antiangiogenesemitteln, Gerinnungshemmern und zellulären Wachstumsfaktoren besteht.

6. Röhrenförmiger vaskulärer Graft gemäss Anspruch 1, worin die Fibrillen parallel zu einer Längsachse des röhrenförmigen Grafts orientiert sind.

7. Röhrenförmiger vaskulärer Graft gemäss Anspruch 1, worin die Knoten in Umfangsrichtung ausgerichtet sind.

## Revendications

1. Greffon vasculaire tubulaire en polytétrafluoroéthylène expansé comprenant :
une surface luminale espacée d'une surface abluminale, lesdites surfaces étant reliées par des noeuds colonnaires cohérents en polytétrafluoroéthylène qui sont séparés par des espaces internodaux colonnaires pré-coagulés ; et
des fibrilles en polytétrafluoroéthylène ayant une longueur moyenne entre 55 et 70 µm entre les surfaces luminale et abluminale, traversant chaque espace internodal colonnaire pré-coagulé pour relier chaque noeud colonnaire à un noeud colonnaire immédiatement adjacent.

2. Greffon vasculaire tubulaire selon la revendication 1 ayant une résistance à la traction radiale d'au moins 0,3 kg/mm².

3. Greffon vasculaire tubulaire selon la revendication 1 ayant une résistance à la traction longitudinale d'au moins 0,9 kg/mm².

4. Greffon vasculaire tubulaire selon la revendication 1, dans lequel les espaces internodaux colonnaires sont au moins partiellement remplis par un matériau non-polytétrafluoroéthylène.

5. Greffon vasculaire tubulaire selon la revendication 4, dans lequel ledit matériau non-polytétrafluoroéthylène est choisi dans le groupe constitué par les antibiotiques, les antimicrobiens, les antinéoplasiques, les inhibiteurs des muscles lisses, les antithrombotiques, les antiplaquettaires, les anti-inflammatoires non stéroïdiens, le collagène, les agents anti-angiogenèse, les anticoagulants, et les facteurs de croissance cellulaire.

6. Greffon vasculaire tubulaire selon la revendication 1, dans lequel les fibrilles sont orientées parallèlement à l'axe longitudinal du greffon tubulaire.

7. Greffon vasculaire tubulaire selon la revendication 1, dans lequel lesdits noeuds sont orientés circonférentiellement.
